Europäisches Patentamt

European Patent Office

Office européen des brevets

(11) Publication number: **0 222 726**

A2

(12) # EUROPEAN PATENT APPLICATION

(21) Application number: 86870164.0

(22) Date of filing: 06.11.86

(51) Int. Cl.⁴: **C 12 N 15/00**
C 12 P 21/02, C 12 N 1/20
A 61 K 37/64
//(C12N1/20, C12R1:19)

(30) Priority: 08.11.85 US 796133

(43) Date of publication of application:
20.05.87 Bulletin 87/21

(84) Designated Contracting States:
AT BE CH DE ES FR GB GR IT LI LU NL SE

(71) Applicant: SMITHKLINE BECKMAN CORPORATION
One Franklin Plaza
Philadelphia Pennsylvania 19103(US)

(72) Inventor: Bollen, Alex Joseph
Gaasbeekstraat, 65
B-1711 Itterbeek(BE)

(72) Inventor: Herzog, Albert Alfred
Elewijtsesteenweg, 190
B-1840 Eppegem(BE)

(72) Inventor: Johansen, Hanne Ranche
649 South Henderson Road
King of Prussia Pennsylvania(US)

(72) Inventor: Rosenberg, Martin
1280 Wisterial Drive
Malvern Pennsylvania(US)

(72) Inventor: Shatzman, Allan Richard
332 Rees Drive
King of Prussia Pennsylvania 19406(US)

(74) Representative: Tasset, Gérard
SMITHKLINE - RIT rue de l'Institut, 89
B-1330 Rixensart(BE)

(54) Mutant coding sequence.

(57) A mutant alpha-1-antitrypsin coding sequence is used to
express a protein having AAT activity at high levels in *E. coli*.

EP 0 222 726 A2

Croydon Printing Company Ltd.

- 1 -

## TITLE

## MUTANT CODING SEQUENCE

## FIELD OF THE INVENTION

This invention relates to an E. coli expression vector having a mutant alpha-1-antitrypsin coding sequence resulting in increased levels of expression of a variant alpha-1-antitrypsin.

## BACKGROUND INFORMATION

Alpha-1-antitrypsin (AAT) is an alpha-1-globulin present in serum and various other body fluids. As synthesized in the liver, mature AAT is a glycoprotein having a molecular weight of about 50,000 to 55,000 daltons. Proteolytic enzymes inhibited by AAT include trypsin, chymotrypsin, pancreatic elastase, skin collagenase, renin and urokinase, as well as proteases of polymorphonuclear lymphocytes. Genetically acquired deficiency of AAT in humans is associated with various pathological conditions, especially emphysema and liver disease. See, for example, Morse, N. Eng. J. Med. 299 (19):1045-1048 (1978) and 299 (20):1099-1105 (1978); Tobin et al., Arch. Int. Med. 143 (7):1342-1348 (1982); and Carrell et al., Nature 298 (5872):329-334 (1982).

Elastase is a proteinase which breaks down lung tissue. Unchecked, its activity can result in emphysema. Gadek et al., Am. Rev. Respir. Dis. 127:S45 (1983) and Glaser et al., Am. Rev. Respir. Dis. 127:547-553 (1983), for example, have shown that AAT can be therapeutically useful in treatment of emphysema.

Because of its therapeutic utility and because comparatively large amounts are required for certain indications, such as replacement therapy for patients genetically deficient in AAT, researchers have been looking for techniques to produce AAT in large quantities. Conventionally, such techniques have involved purification of AAT from blood plasma. See, for example, Coan et al., U.S. Patent 4,379,087.

Courtney et al., EP-A-114,777, disclose expression of an AAT fusion protein, lacking the N-terminal glutamic acid residue of AAT, in E. coli. Courtney et al. report that expression of unfused AAT in E. coli is very low.

Courtney et al., Nature 313:149 (1985) report synthesis in E. coli of a variant AAT having an active site mutation.

Bollen et al., Belgian Patent 895,961, disclose cloning of AAT in E. coli on a plasmid, pULB1523, comprising a 1.4 kilobase pair (kb) fragment of cDNA reversely transcribed from polyA-RNA derived from human liver cells cloned into the PstI site of pBR322. Bollen et al. disclose that the cDNA fragment comprises the entire AAT gene, including the native translation initiation codon.

Bollen et al., DNA 2:255 (1983) disclose pULB 1523 and pKT287, an expression vector which results in expression of a beta-lactamase-AAT fusion protein in E. coli.

Bollen et al., FEBS Letters 116(1):67-70 (1984), report expression of AAT in E. coli using the lambda

- 3 -

0222726

promoter, PR, and reports that attempts to demonstrate biological activity of E. coli-derived AAT have been unsuccessful.

Parker et al., Australian Patent Application 31801/84, disclose expression of AAT in E. coli.

Munson et al., J. Microbiol. 177:663-683 (1984), report mutations of the E. coli lacZ gene which result in increased expression of beta-galactosidase. Myers et al., Science 229:242 (1985), report an electrophoresis procedure for identifying mutant DNA molecules in an absence of phenotypic selection.

## SUMMARY OF THE INVENTION

In one aspect, the invention is a recombinant DNA molecule comprising a mutant AAT coding sequence wherein the mutation comprises an alteration of the AAT coding sequence within codons 1-15, provided that the mutation is other than or additional to substitution of the codon ATG for the first codon of the native coding sequence.

In other aspects, the invention is an E. coli expression vector having the DNA molecule operatively inserted therein and an E. coli transformed with the expression vector of the invention. The invention is also a method of producing a polypeptide having the biological activity of AAT, that is, anti-trypsin and anti-elastase activity, which comprises culturing the transformed E. coli of the invention under permissive conditions and isolating the AAT so produced.

In other aspects, the invention is a variant AAT protein having the biological activity of AAT and having an altered amino acid sequence within positions 1-15, provided that the alteration is other than or additional to substitution of the glutamic acid residue in the first position of mature AAT; a pharmaceutical composition for inhibiting elastase activity in the lungs of an animal, including a human, comprising the variant AAT protein and a pharmaceutically acceptable carrier; and a method for inhibiting elastase activity in the lungs of an animal, including a human, which comprises administering internally to the animal an anti-elastase effective amount of the variant AAT protein.

In yet another aspect, this invention comprises a method of identifying a mutant coding sequence for a polypeptide of interest which is transcribed and translated in an amount greater than the native coding sequence which comprises (i) ligating the whole or a portion of the native coding sequence to a coding sequence for a selectable phenotype, transforming a suitable host therewith and quantitating expression of the resulting translational fusion, (ii) mutagenizing the whole or the portion of the coding sequence for the polypeptide of interest, (iii) assaying for increased expression of the mutant translational fusion over the expression of the original translational fusion; (iv) determining the nucleotide sequence of the mutant portion of the coding sequence for the polypeptide of interest in fusions displaying increased expression in (iii) and selecting those having a mutation in the coding sequence for the polypeptide of interest, (v) preparing a coding sequence identical to the native coding sequence of the product of interest except that it contains one or more of the mutation(s) identified in the mutant coding sequence and transforming a suitable host therewith, and (vi) selecting clones of transformants from (v) in which the mutant coding sequence is transcribed and translated in amounts greater than the native coding sequence.

## DETAILED DESCRIPTION OF THE INVENTION

It has now been found that expression of active AAT in bacteria can be significantly and unexpectedly improved by altering the coding sequence for the N-terminus of mature AAT without destroying the biological activity of the protein, that is, without destroying proteinase, especially elastase, inhibition by the protein. Using the recombinant DNA molecule of the invention, a variant AAT protein having anti-trypsin and anti-elastase activity can be expressed in bacteria in amounts 2-fold or more greater than amounts of native AAT. In preferred embodiments of the invention, the amounts of the variant AAT expressed can be 100-fold or more greater than native AAT. Native AAT or AAT, as used herein, means mature AAT, as shown in Table 1, below, which has Glu-Asp-Pro as the N-terminal amino acids as well as the Met-Asp-Pro construct obtained by cleaving the native coding sequence at the BamHI site between Glu and Asp. See Ex. 2 below. Variant AAT, as used herein, has a primary amino acid sequence different than that of native AAT. A mutant AAT coding sequence is one which differs from the coding sequence for native AAT shown in Table 1, below. The recombinant DNA molecule of the invention, thus, is such mutant coding sequence, either isolated or within a larger DNA molecule such as, for example, a cloning or expression vector. The recombinant DNA molecule of the invention is prepared by standard synthetic or genetic engineering techniques, or by a combination of both, as explained hereinbelow.

The coding sequence comprised within the recombinant DNA molecule of this invention has one or more substitutions of base pairs which do not affect amino acid sequence, and/or substitution, addition or deletion of one or more codons, within the first fifteen codons. The variant AAT coding sequences within exemplary recombinant DNA molecules of the invention, construction and use of which are exemplified herein, are illustrated in Table 1, which follows, as are the first twenty codons of native AAT for comparison. Variant base pairs are underscored.

Table 1

| Construct | Coding sequence (5′ → 3′) |
|---|---|
| Native | AAT — Glu GAG · Asp GAT · Pro CCC · Gln CAG · Gly GGA · Asp GAT · Ala GCT · Ala GCC · Gln CAG · Lys AAG · Thr ACA · Asp GAT · Thr ACA · Ser TCC · His CAC · His CAT · Asp GAT |
| pHJ₁-2 | Met ATG · Glu GAA · Asp GAT · Pro CCG · Gln CAG · Gly GGT · Asp GAC · Ala GCT · Ala GCG · Gln CAG · Lys AAA · Thr ACC · Asp GAC · Thr ACT · Ser TCC · His GAC · His CAT · Asp GAT · Gln CAG · Asp GAT · His CAC...3′ |
| pHJ₁-3 | Met ATG · Asp GAT · Pro CCG · Gln CAG · Gly GGT · Asp GAC · Ala GCT · Ala GCG · Gln CAG · Lys AAA · Thr ACC · Asp GAC · Ser TCC · His CAC · His CAT · Asp GAT · Gln CAG · Asp GAT · His CAC...3′ |
| pHJ₁-4 | Met ATG · Glu GAA · Asp GAT · Pro CCG · Gln CAG · Gly GGT · Asp GAC · Ala GCT · Ala GCG · Gln CAG · Lys AAA · Thr ACC · Asp GAC · Thr ACT · Ser TCC · His CAC · His CAT · Asp GAT · Gln CAG · Asp GAT · His CAC...3′ |
| pHJ₁-5 | Met ATG · Asp GAT · Thr ACA · Ser TCC · His CAC · Asp GAT · Gln CAG · His CAT · His CAC...3′ |
| pHJ₁-6 | Met ATG · Asp GAT · Ala GCT · Thr ACA · His CAT · Lys AAG · Thr ACA · Asp GAT · Ser TCC · Gln CAG · Thr ACA · Gln CAG · His CAT · Asp GAT · His CAT · Asp GAT |
| Syn 7 | Met ATG · Asp GAT · Pro CCG · Gln CAA · Gly GGT · Asp GAT · Ala GCC · Gln CAG · Lys AAG · Thr ACA · Ser TCC · His CAC · His CAT · Asp GAT · His CAC...3′ |

Table 1 (cont'd)

Syn 7R
Met Asp Pro Gln Gly Asp Ala Gln Lys Thr Asp Thr Ser His His Asp Gln
ATG GAT CCG CAA GGT GAT GCC CAG AAG ACA GAT ACA TCC CAC CAT GAT CAG
                                              Asp His
                                              GAT CAC...3'

pUTS₃₁Bcl
Met Asp Gln Asp His
ATG GAT CAG GAT CAC...3'

p 1-A
Met Asn Pro Gln Gly Asp Ala Ala Gln Lys Thr Asp Thr Ser His His Asp
ATG AAT CCC CAG GGA GAT GCT GCC CAG AAG ACA GAT ACA TCC CAC CAT GAT
                                              Gln Asp His
                                              CAG GAT CAC...3'

p 1-AG
Met Asn Pro Gln Gly Asp Ala Ala Gln Lys Thr Asp Thr Ser His His Asp
ATG AAT CCG CAG GGA GAT GCT GCC CAG AAG ACA GAT ACA TCC CAC CAT GAT
                                              Gln Asp His
                                              CAG GAT CAC...3'

pH 1-7
Met Asn Pro Gln Gly Asp Ala Ala Gln Lys Thr Asp Thr Ser His His Asp
ATG AAT CCG CAA GGT GAT GCT GCC CAG AAG ACA GAT ACA TCC CAC CAT GAT
                                              Gln Asp His
                                              CAG GAT CAC...3'

0222726

The amounts of AAT or variant AAT expressed in E. coli by recombinant DNA molecules of the invention in terms of percentage of total cellular protein are shown in Table 2, which follows. All of the listed plasmid expression vectors, except those bearing the designation "cro", employ the leftward promoter (PL) of lambda and the cII ribosome binding site. The cro vectors employ PL and the cro ribosome binding site. The plasmid constructions are described in the Examples, below. The reported representative expression data were obtained by induction with naladixic acid or, in the case of data in parentheses, by heat induction. See, Mott et al., Proc. Natl. Acad. Sci. U.S.A. 82:88 (1985). Table 2 also shows the designations given to the product of each vector.

## Table 2

| Coding Sequence | Protein | % of Total Cellular Protein |
|---|---|---|
| $pOTS_{\alpha l}$ | AAT | 0.1-0.3 |
| $pHJ_{\alpha l}-1$ | AAT | (0.1-0.3) |
| $pHJ_{\alpha l}-2$ | HJ2 | (0.3-0.5) |
| $pOTS_{\alpha l}-3$ | AAT | 0.3-0.5 |
| pSyn7 | Syn7 | 1-2(.3-.5) |
| pSyn7R | AAT | 1-2(.3-.5) |
| $pOTS_{\alpha l}Bcl$ | AATD15 | 20-30 |
| $pHJ_{\alpha l}-4$ | HJ2 | (0.5-1) |
| $pHJ_{\alpha l}-5$ | AATD10 | (4-6) |
| $pOTS_{\alpha l}-5$ | AATD10 | 13-18 |
| $pHJ_{\alpha l}-6$ | AATD5 | (3-5) |
| $pOTS_{\alpha l}-6$ | AATD5 | 10-15 |
| $P_{\alpha l}-A$ | HJ7 | 1-2 |
| $P_{\alpha l}-Acro$ | HJ7 | 3-5 |
| $P_{\alpha l}-AG$ | HJ7 | 2-3 |
| $P_{\alpha l}-AGcro$ | HJ7 | 5-10 |
| $pHJ_{\alpha l}-7$ | HJ7 | (3-5) |
| $pOTS_{\alpha l}-7$ | HJ7 | 10-15 |

When heat induction is employed (see, e.g., M. Rosenberg et al., Meth. Enzymol. 101:123-138 (1983), AAT production is approximately one-third of that achieved by naladixic acid induction. For example, using pOTS $_{\alpha1}$-Bcl, variant AAT expression was 5-10% of total cellular protein; using p$_{\alpha1}$-AGcro, it was 2-4%; and using pHJ$_{\alpha1}$-7, it was 3-5%.

The recombinant DNA molecules shown above are illustrative only. Alternative or additional substitutions of base pairs and deletions of codons can be made by techniques well known in the field of molecular genetics. The effect of such mutations on expression levels in bacteria can be readily determined by well known techniques such as by ELISA as disclosed, for example, by Bollen et al., DNA 2:255 (1983). Activity of resulting proteins can be readily assayed in well known screens, such as for elastase and trypsin inhibition as disclosed by Courtney et al., EP-A-114,777; Kawasaki et al., EP-A-103,409; and Travis, U.S. Patent 4,493,891. Other illustrative DNA molecules of the invention are such molecules wherein the mutant coding sequence differs from that of native AAT by substitution of one or more base pairs causing a change in the amino acid sequence of AAT.

Also included within the invention are recombinant DNA molecules wherein the AAT coding sequence comprises mutations additional to mutations in the 5' end of the AAT coding sequence. For example, the coding sequence can be fused at the 5' and/or 3' end to a coding sequence for one or more additional amino acids or polypeptides, such as a N-terminal Met fusion as in pHJ$_{\alpha1}$-2 and -4, or fusions such as disclosed by Courtney et al., EP-A-114,777 or Bollen et al., DNA 2:255 (1983). Fusion of a N-terminal portion of the influenza virus NS1 coding sequence to authentic AAT increases expression of the AAT hybrid. The coding sequences also can comprise substitutions of base pairs or deletions of codons within regions of the coding sequence other than the 5' end. Excluded from the invention, of course, are mutations which result in lowering of expression levels over those obtained with the native coding sequence, e.g., expression in E. coli at less than about 0.3%, of total cellular protein, or loss of biological activity of the resulting protein, such as, frameshift mutations, nonsense mutations or active site mutations which interfere with activity. Exemplary of acceptable active site mutations is a change of the methionine amino acid at position 358 to a valine residue, such as disclosed by Courtney et al., Nature 313:149 (1985) and by S. Rosenberg et al., Nature 312:1 (1984). The variant AAT of the invention can also be bound or complexed with other molecules such as disclosed, e.g., by Mitra, U.S. Patent 4,496,689.

As noted previously, mutations to the 5' end of the AAT coding sequence, as well as to other regions, can be made by well known techniques. For a review of certain such useful techniques, see, Botstein et al., Science 229: 1193(1985). These include, for example, deletion of codons by treatment of the coding sequence with a restriction endonuclease optionally followed by treatment with an exonuclease, e.g., Bal31 or mung bean nuclease. Following such deletion, synthetic sequences can be inserted to replace all or part of the deleted region. Alternatively, mutations can be induced, for example, by treatment of transformants carrying an AAT coding sequence with chemical mutagens or irradiation. Mutants altered in the 5' region can be selected by expression of AAT and nucleotide sequencing of the first twenty codons. Such mutagenesis experiments are conveniently carried out by fusing N-terminal AAT coding sequences, e.g., the 45 base pair BamHI-BclI fragment, upstream of a gene for a selectable phenotype, e.g., galactokinase and beta-galactosidase, and screening for up-mutations, that is, mutants exhibiting increased levels of expression of the gene for the selectable phenotype.

Thus, another aspect of this invention is a method for identifying mutants of a coding sequence for any polypeptide of interest which mutants are transcribed and translated at levels greater than that of the native coding sequence. This method can be carried out by ligating the whole or a portion of the native coding sequence for the product of interest to the 5' or 3' terminus of a coding sequence for a readily selectable function. Preferably, the portion of the native coding sequence will be a portion from the 5' end, for example, 45 base pairs, which is fused to the 5' terminus of the coding sequence for the selectable function.

The selectable function is any function which confers a selectable phenotype and is preferably one whose expression can be relatively quantitated. For example, the selectable function can be galactokinase activity, for which relative expression can be quantitated by color change on an indicator plate, e.g., MacConkey galactose (Difco). Galactokinase translational fusions are described, for example, by Heusterspreute et al., DNA 3:377-386 (1984). Other examples include beta-galactosidase activity which can be relatively quantitated by color change on an indicator plate comprising a nutrient agar containing X-Gal or ONPG (See, for example, J.H. Miller, Experiments in Molecular Biology, Cold Spring Harbor Laboratory, New York, 1972.

Such translational fusions are then mutagenized, such as, for example, by passage through a mutator strain or by treatment in vitro with a mutagen such as hydroxyl-amine as disclosed by Munson et al., J. Microbiol. 177:

663-683 (1984). See, also, J.H. Miller, Experiments in Molecular Biology, Cold Spring Harbor Laboratory, New York, 1972. Mutant transformants or hosts transformed with the mutated plasmids are cloned and screened for increased expression of the selectable function. Plasmids from clones which exhibit such increased expression are then isolated and analyzed by DNA sequencing of the portion coding for the polypeptide of interest. Some of these plasmids are mutated in the coding sequence for the product of interest. Mutations in this portion are then inserted into the entire coding sequence for the product of interest by standard synthesis/recombinant DNA techniques. Such mutant coding sequences are inserted into an expression vector and introduced into a host cell. The transformants are cloned and assayed for expression of the polypeptide of interest. In some cases, it will be found that unrelated mutations in different plasmids cause increased expression. Such mutations can be combined into a single variant coding sequence as in the case of $pHJ_{\alpha 1}$-7. See, Example 9, below.

The expression vector into which the mutant AAT coding sequence is inserted to prepare the vector of the invention can be any of the numerous bacterial expression vectors known and available in the field. Regulatable expression vectors are preferred. By "regulatable" is meant that transcription of the inserted coding sequence is not constitutive, but can be induced, such as by addition to the culture medium of an inducing agent or by heat. Exemplary E coli. expression vectors include, among others, pCQV2, described by Bollen et al., FEBS Lett. 166-67 (1984), and pAS1, described by M. Rosenberg et al., Meth. Enzym. 101:123-138 (1983). pAS1 carries the pBR322 origin of replication, an ampicillin resistance marker and a series of fragments from lambda which comprise the regulatory region, namely, the leftward promoter of lambda
(PL), N anti-termination function recognition sites (NutL
and NutR), the rho-dependent transcription termination
signal (tRl) and the cII ribosome binding site, including
the cII translation initiation site, the G residue of
which is followed immediately by a BamHI cleavage site as
follows:

5'..... CATATG*GATCC ......3' wherein the symbol, *, represents the cleavage site for
BamHI.

pAS1 can be derived from pKC30cII by deleting
nucleotides from the BamHI site at the cII-pBR322 junction
of pKC30cII to the cII ATG and religating the molecule to
regenerate the BamHI site immediately downstream of the
ATG.  pKC30cII is constructed by inserting a 1.3 kb HaeIII
fragment from lambda which carries the cII gene into the
BpaI site of pKC30.  See, Shatzman et al., in
"Experimental Manipulation of Gene Expression," Edit. by
M. Inouye, Academic Pres, New York (1983) and M. Rosenberg
et al., cited above.  pKC30 is described by Shimitake et
al., Nature 292:128 (1981).  It is a pBR322 derivative
having a 2.4 kb HindIII-BamHI fragment of lambda inserted
between the HindIII and BamHI sites in the tetR gene of
pBR322.  Constructions similar to pAS1 are described by
Courtney et al., Nature 313:149 (1985) and Kotewicz et
al., Gene 35:249 (1985).  The coding sequence is
operatively inserted therein, that is, in correct
orientation and in proper reading frame to the regulatory
element, by standard techniques.

Other cloning and expression systems are known
and available for expressing the mutant AAT coding
sequence of the invention in other bacteria, e.g.,
Bacillus, Streptomyces, Corynebacterium and others.

A coding sequence for mature, native AAT, of which
any of the naturally occuring polymorphs can be used as a

- 14 -

0222726

starting material for carrying out this invention, can be obtained by standard techniques by reverse transcription of selected messenger RNA populations from liver cells such as disclosed, for example, by Bollen et al., Belgian Patent No. 895,961; Bollen et al., _DNA_ 3:255 (1983); Courtney et al., EP-A-114,777; Kawasaki et al., "The Molecular Biology of Yeast," August 16-21, 1983, Cold Spring Harbor Laboratory; Long et al., Structure and Organization of Genes I, Abstract No. 25 (1983); Woo et al., "From Gene to Protein: Translation into Biotechnology," edit. by Ahmed et al., Academic Press, New York (1982); Kurachi et al; _Proc. Nat'l. Acad. Sci. U.S.A._ _78_: 6826(1981); Parker et al., Australian Patent Application 31801/84; and, Costanzo et al., _EMBO J._ 2:57(1983), all of which are incorporated by reference herein as though fully set forth.

Variant AAT is produced in accordance with the process of the invention by culturing microorganisms or cells which have been transformed with the expression vector of the invention under permissive conditions. By "permissive conditions" is meant culture conditions, e.g., analytes, metabolites, other medium ingredients and temperature, under which expression of AAT is induced. Typically, transformed _E. coli_ are cultured in a nutrient broth containing assimilable sources of carbon and nitrogen, with aeration and under selection pressure, until log phase growth ($A_{650}$ about 0.4-0.6) prior to induction and then for an additional 1-1/2 to 5 hours following induction until a recoverable quantity of the polypeptide is expressed.

The variant AAT of the invention expressed in _E. coli_ or other organism is isolated from the producing culture by standard protein isolation techniques. Typically, the purification scheme comprises 1) disruption of cells, 2) clarification of the cellular extract, 3) separation of the variant AAT molecules and 4) final purification to remove residual contaminants including residual polypeptides, carbohydrates, nucleic acids and/or lipopolysaccharides.

The first step can be accomplished such as by addition of lysozyme or other lysing or permeabilizing agent or by mechanical or ultrasonic disruption. Other means for externalizing the protein include use of temperature-sensitive lytic bacteria such as disclosed by Auerbach and Rosenberg, European Patent Application Publication No. 0140864. If the polypeptide is precipitated within the cell extract, it can be resolubilized by addition of a denaturant. Such techniques are well known. Several of these are reviewed by Builder et al., EP-A-114,506, published August 1, 1984. The variant AAT molecules specifically exemplified herein are soluble in standard E. coli extracts, with the exception of AATD15.

The variant AAT molecule in solution is then separated by standard techniques. These include, for example, selective precipitation of the variant AAT such as by addition of ammonium sulfate (60 to 80% of saturation) followed by resolubilization. The variant AAT-containing solution can then be passed through a series of one or more chromatographic steps, such as using DEAE-cellulose, concanavalin A sepharose, phenylsepharose, disulfide binding ligands, reverse phase HPLC, and affinity columns using anti-AAT antibody, preparation of which is disclosed by Lathe et al., Nature 284:473-474 (1980). The purified variant AAT can be lyophilized prior to resolubilization in a pharmaceutically acceptable diluent for administration.

The variant AAT molecule tends to be unstable below about pH 5.5. The proteins are stable for 1-2 days at room temperature and for considerably longer at 4°C.

For storage of purified AAT, sodium phosphate buffer (pH 8) is typically employed.

The preferred purification process is a substitution chromatography process which comprises contacting an impure solution of a variant AAT protein of the invention with Kappa (K)-chains previously coupled to an insoluble carrier matrix. See, Laurell et al., Eur. J. Biochem. 57:107-113 (1975); Laurell, J. Chromat. 159:25-31 (1978); Laurell, J. Chromat. 278:53-61 (1983). The K-chains can be derived from urine of a patient with myelomatopathy and can be bound to a suitable carrier matrix by known techniques. Carrier matrices include, for example, agarose, Affi-gel®, dextran, cellulose, polystyrene, polyacrylamide, silica, nylon, acrylic copolymers and polyesters.

The variant AAT of the invention is useful as a protease inhibitor and can be used in the treatment of emphysema caused by insufficient AAT activity. Such insufficiency can be caused by oxidation of AAT, such as by tobacco smoke, or by genetic deficiency of AAT production. Gadek et al., Am. Rev. Respir. Dis. 127:545 (1983), for example, demonstrated the usefulness of AAT in parenteral replacement therapy of homozygous AAT deficient patients. Other uses for the variant AAT include antibody production, use as a binding agent in chromatographic purification of various proteins and protease inhibition in protein containing mixtures or solutions. Yet other uses include treatment of pancreatitis and burns (see, Schultze et al., U.S. Patent 3,293,236) and for production of antibodies for use in diagnosing serum AAT-deficient individuals. Such uses can be accomplished by techniques which are well within the ordinary skill in the respective art.

The variant AAT of the invention has AAT activity and is immunologically closely related to AAT. It can be used, therefore, in the same manner as AAT. Specifically, the variant AAT can be formulated, such as by dissolution in a pharmaceutically acceptable carrier, by standard techniques for internal administration, typically, intravenous administration. Pharmaceutically acceptable carriers are well known. Such carriers which are suitable for intravenous administration include, for example, solutions of sodium chloride, acetates, glucose, mannitol and albumin. The variant AAT can also be complexed with physiologically acceptable excipients such as disclosed by Mitra, U.S. Patent 4,496,689. The dosage and frequency of administration will vary with the nature and extent of the disease and with the activity of the particular variant molecule employed. In a patient suffering from a homozygotic AAT deficiency, the dosage and frequency of administration are selected to maintain serum AAT activity at levels approximating at least 35% of normal. Typically, this requires i.v. infusion of 1-15 g, preferably 2-10 g of the variant AAT, having activity comparable to AAT, weekly. Variant molecules which, by virtue of mutations in other regions of the protein, such as in the active site, exhibit more stable activity, may be administered in lower doses and/or with lower frequency.

The following examples are illustrative, and not limiting, of the invention. All restriction endonucleases were obtained from commercial sources and were used substantially in accordance with the vendors' instructions.

## EXAMPLES

### Ex. 1. pOTS $_{\alpha 1}$ Bcl

The human AAT gene was isolated from the cDNA clone pULB1523 (obtained from A. Bollen, Belgian Patent 895,961). The gene was obtained on a PstI fragment encompassing the entire cDNA clone.

The PstI fragment was inserted into a unique PstI site in pUC9 (a pBR322 vector obtained from Bethesda Research Laboratories, Gaithersburg, Maryland, containing a fragment from M13 with multiple unique sites). This new vector was digested with endonuclease SalI, which resulted in a unique cut positioned 10 bp downstream of the PstI site at the 3' end of the cDNA fragment. The linearized vector was treated with exonuclease Bal 31 to remove the GC tailing following the 3' end of the AAT gene. Conditions were as follows: 0.4 mM $CaCl_2$, 0.4 mM $MgCl_2$, 40 mM NaCl, 24 mM Tris pH8, 0.2 mM EDTA and 40 mg of DNA in a total volume of 200 ml. The reaction was incubated at 30°C for 2 min and 20 units of exonuclease Bal31 (obtained from Bethesda Research Laboratories, Gaithersburg, Maryland) was added. 25 ml aliquots were removed from the mixture every 15 sec and the reaction was stopped by adding EDTA to a final concentration of 50 mM. Analysis of the DNA from the 1 min timepoint showed that the GC tailing was removed from the 3' end of the AAT gene and that the gene had retained 40 base pairs downstream of the stop codon for AAT. The DNA was filled in using Klenow DNA polymerase (Maniatis et al. Molecular Cloning: A laboratory manual, 1982, Cold Spring Harbor Laboratory, Cold Spring Harbor, New York) creating a blunt end molecule and an XhoI linker obtained from Collaborative Research Laboratories, Lexington, Massachusetts, was inserted (Maniatis et al.) resulting in the construction $pUC_{\alpha 1}-2$.

The AAT gene was isolated from $pUC_{\alpha 1}-2$ by digestion with XhoI and BclI Belt cuts 45 bp downstream of the initiation codon at the 5' end of the AAT gene. The gene was inserted into the expression vector pOTSV which had been digested with BamHI and XhoI, using standard ligation procedures (Maniatis et al.). The resulting construct, $pOTS_{\alpha 1}Bcl$, is an expression vector of the invention. It contains a variant AAT gene that codes for the entire AAT minus the first 15 amino acids of the mature 5' end of the gene.

pOTSV (Devare et al., Cell 36:43-49 (1984)) was derived from pAS1 by inserting a 189 bp fragment carrying a transcription terminator, the OOP terminator, into the NruI site downstream of the BamHI site in pAS1 and by inserting a synthetic linker (XbaI, XhoI, SacI) into the SalI site between the BamHI site and the added terminator.

A lysogenic htpR165 strain of E. coli (Cooper et al., Mol. Gen. Genet. 139:167 (1975); Baker et al., Proc. Natl. Acad. Sci. U.S.A. 81:6779 (1984) and a wild-type, $cI^+$ defective K12 lysogen were transformed with pOTS $_{\alpha 1}$Bcl by standard procedures (Maniatis et al.). Transformants were grown to log phase prior to heat induction (M. Rosenberg et al., Meth. Enzym. 101:123 (1983)) or naladixic acid induction (Mott et al., Proc. Natl. Acad. Sci. U.S.A. 82:88 (1985)).

Following the induction period, cells were collected by centrifugation and lysed by addition to a lysozyme solution (1 g cells/10 ml solution) 50 mM Tris, pH8, 0.1 mM ethylenediaminetetraacetate (EDTA), 0.1 mM dithiothreitol (DTT), 0.1% deoxycholate (DOC) and 0.5 g/ml of lysozyme). The suspension was sheared by sonication and then stirred for at least 4 hours. The suspension was then separated by low-speed (10,000 rpm) centrifugation for 20 min. The pellet was resuspended in the Tris buffer to which urea was previously added to a final concentration of 4M. This suspension was sonicated and stirred overnight at room temperature. The suspension was then separated by low-speed centrifugation for 20 min. The supernatant was collected and dialyzed against buffer (50 mM Tris, pH 8, 25 mM NaCl, 1 mM DTT). The solution was then loaded onto a diethylaminoethyl (DEAE)- Trisacryl column and eluted with 25-500 mM NaCl gradient. Peak fractions were dialyzed against buffer (50 mM Tris, pH 8, 25 mM NaCl) and assayed for protein concentration and anti-trypsin and anti-elastase activity. Fractions collected at 150-250 mM NaCl contained AATD15 at about 90% purity.

In the trypsin and elastase assays, AATD15 exhibited 10-15% of the activity of AAT derived from human serum (Sigma Chemicals, St. Louis, Missouri) and of AAT (Met-Asp-Pro) derived from yeast. This lessening of activity is believed to have resulted from use of urea and, in another case, thiocyanate, under alkaline conditions. The trypsin assay was carried out by the procedure of Travis, Meth. Enzym. 80:755-765 (1981). A reaction mixture of 100 ml of trypsin (15 mg), 200 ml of Tris (10 mM, pH 8) and 200 ml of AATD15 (30 mg) in 10 mM sodium phosphate (pH 7.6) was incubated for 15 min at room temperature. Then, 2.5 ml of substrate (p-tosyl-L-arginine methyl ester (1.3 mg/ml) in buffer (10 mM Tris, pH 8) was added. Absorbance at 253 nm was plotted each minute for a 5 minute period.

The elastase assay was also by the procedure of Travis. A stock substrate solution (20 mM) was prepared by adding 45 mg of N-succinyl-L-alanyl-L-alanyl-L-alanine-p-nitroanilide (Sigma Chemical Co., St. Louis, MO) to 5 ml of dimethylsulfoxide (DMSO). An enzyme solution was prepared by adding 0.3 mg of elastase from porcine pancreas (Sigma Chemical Co., St. Louis, MO). To 250 ml of the enzyme solution in a cuvette were added 0.2 ml of buffer (200 mM Tris-HCl, pH 8). An equimolar amount of AATD15 was added to the solution and the solution was incubated at room temperature for 1 min. Deionized water was added to a final volume of 2,950 ml. Then, 50 ml of the substrate solution was added and the solution was mixed. Absorbance of 410 nm was observed for a 5 minute period.

## Ex. 2. pHJ $\alpha_1$-2

pMG27N (Gross et al., Mol. Cell. Biol. 5:1015 (1985)) was derived from pOTSV by deleting a EcoRI-BalI fragment of about 900 bp upstream of the PL promoter, resulting in deletion of 3 of the 4 NdeI sites in the vector. A BamHI-XhoI (end-filled) fragment of the AAT coding sequence from pULB1523 (BamHI cuts the coding sequence between the first and second codons) was inserted between the BamHI and SalI sites in pMG27N. The procedures were carried out substantially as described by Maniatis et al., cited above. This vector, pHJ $\alpha_1$-1, expressed mature, native AAT (having a Met in place of Glu as the first amino acid) as less than about 0.1-0.3% of total cellular protein. The BamHI site downstream of the cII ATG is retained.

pHJ $\alpha_1$-1 was digested with NdeI and BclI to delete codons two through sixteen. This fragment was replaced with a synthetic oligonucleotide linker, as illustrated in Table 1, to prepare pHJ $\alpha_1$-2. The fragments were inserted by using a plasmid to oligonucleotide ratio of 1:50 and standard ligation procedures. The variant AAT protein, HJ2, was expressed in an amount about 2-fold greater than AAT from pHJ $\alpha_1$-1, following heat or naladixic acid inductions, as estimated by scanning of polyacrylamide gels stained with Coomassie Brilliant Blue.

## Ex. 3 pOTS $\alpha_1$-3

The entire AAT gene was isolated from pUC $\alpha_1$-2 by digestion with BamHI and XhoI. The fragment was ligated to a BamHI and XhoI cut pOTSV vector resulting in pOTS $\alpha_1$. pOTS $\alpha_1$ was deposited in the American Type Culture Collection, Rockville, Maryland, U.S.A., under accession number 53282. This new vector was then digested with BamHI and BclI which excises the DNA fragment from the N-terminal end of AAT containing the first 15 amino acids of the mature protein. This fragment was replaced by synthetic DNA (i.e., two overlapping oligonucleotide linkers) coding for these 15 amino acids as illustrated in Table 1, above. This vector, pOTS $_{\alpha 1}$-3, expresses AAT at levels similar to the variant AAT produced by pHJ $_{\alpha 1}$-2.

### Ex. 4.  pSyn7

A BamHI - PstI (end-filled) fragment from the original AAT cDNA clone, pULB1523, was inserted into pAS1 digested with BamHI and SalI (end-filled). The inserted AAT fragment contains the GC tail at its 3' end. A BamHI - Fnu4HI fragment at the 5' end of the AAT gene was replaced with a synthetic oligonucleotide linker wherein 3rd position codon changes were introduced into 3 codons. This construction codes for a full-length AAT wherein the N-terminal amino acid sequence is met-asp-pro-, as illustrated in Table 1, above. When the DNA sequence was analyzed, it was found that the 7th codon was deleted during construction. Thus, in addition to the 3rd position codon changes, this AAT construct deletes amino acid 7.

The Syn 7 protein was purified by ammonium sulfate precipitation. Specifically, transformants were collected by centrifugation and lysed for 30 min at 0°C in one-tenth volume of TES buffer (50 mM Tris, pH 8, 5 mM EDTA and 25% sucrose) supplemented with 0.5 mg/ml of lysozyme and 10 mg/ml of DNAse. Triton X100 was then added to a final concentration of 0.1%. The suspension was incubated at 0°C for 15 min and then brought to 80 mM Mg$^{++}$ by addition of 1M MgCl$_2$. This suspension was incubated for 5 min at 25°C. A supernatant was collected following centrifugation at 15,000 rpm for 10 min. Ammonium sulfate was added to the supernatant and the solution was centrifuged for 10 min at 10,000 rpm. The Syn 7 protein was detected in the 50-75% ammonium sulfate fraction. This precipitate was resolubilized in buffer (50 mM Tris, pH 8, 25 mM NaCl) and dialyzed against the same buffer. The solution was loaded onto a DEAE-Trisacryl column and eluted with a 25-500 mM NaCl gradient in buffer (50 mM Tris, pH 8). Fractions were dialyzed against buffer (10 mM sodium phosphate, 150 mM NaCl) and protein concentration and anti-trypsin and anti-elastase activities were assayed substantially as described in Ex. 1, above. The Syn 7 protein was found in the 150 - 250 mM NaCl fractions at less than 25% purity. It had the same level of anti-elastase and anti-trypsin activities as serum-derived and yeast-derived AAT. The Syn 7 construct produced about 7-fold more protein than pHJ $\alpha_1$-1.

In a separate experiment, Syn 7 was purified to greater than 95% substantially by the procedure of Laurell et al., J. Chromatog. 278:53-61 (1981). See, also, Laurell et al., Eur. J. Biochem. 57:107-113 (1975) and Laurell, J. Chromatog. 159:25-31 (1978). Monomeric Kappa chains isolated from urine of a patient with myelomatopathy substantially by the procedure of Berggard et al., J. Biol. Chem. 244:4299-4307 (1969) were coupled to cyanogen bromide sepharose 4B (Pharmacia, Piscataway, New Jersey) substantially by the procedure of Laurell et al., Eur. J. Biochem. 57:107-113 (1975). A buffered solution of Syn 7 (50 mM Tromethamine) partially purified from cell lysates by ammonium sulfate precipitation was reduced by addition of beta-mercaptoethanol to a final concentration of 20 mM. The solution was then applied to a Biogel P30 molecular sieve. The solution was then passed through the K-chain/sepharose column and eluted with the same buffer modified by addition of 1 mg/ml of dithiobisnitrobenzoic acid and 0.25 mg/ml of dithiothreitol. The eluate was reduced by addition of β-mercaptoethanol to a final concentration of 20 mM and dialyzed or applied to a Biogel P150 molecular sieve in sodium phosphate buffer (10 mM, pH 7.5, 0.15 M NaCl).

## Ex. 5.  pSyn7R

pSyn7R was constructed as described in Example 4, above.  It was confirmed by sequence analysis that this construction codes for a full length AAT where the N-terminal amino acid sequence is met-asp-pro-, as illustrated in Table I.  The expression of AAT from pSyn7R was about the same as for Syn7.

## Ex. 6.  pHJ $_{\alpha 1}$-5 and pOTS $_{\alpha 1}$-5

pHJ $_{\alpha 1}$-1 was digested with NdeI and BclI which deleted a fragment consisting of the first 15 amino acids of the N-terminal end of AAT.  This fragment was replaced with a synthetic DNA sequence coding for the last five of these amino acids.  The final construction coded for entire AAT minus the first 10 amino acids of the mature 5' end of the gene.  The protein, herein referred to as AATD10, was purified substantially as described in Example 4, above. It was shown to be 100% active in the trypsin and elastase assays as described in Example 1 and was expressed in an amount about 200- to 250-fold greater than AAT from pHJ $_{\alpha 1}$-1.  A BglII-XhoI fragment from this vector was cloned into pOTS $_{\alpha 1}$ between BglII-XhoI to construct pOTS $_{\alpha 1}$-5.

## Ex. 7.  pHJ $_{\alpha 1}$-6

pHJ $_{\alpha 1}$-6, which expresses AATD5, was constructed and the protein was purified therefrom substantially as described in Ex. 6, above.  The amount of AATD5 was about 150- to 200-fold the amount expressed by pHJ $_{\alpha 1}$-1. AATD5 was tested in the trypsin assay substantially as described in Ex. 4 above, and found to be 100% active. A BglII-XhoI fragment was inserted into pOTS $_{\alpha 1}$ as in Example 6 to construct pOTS $_{\alpha 1}$-6.

## Ex. 8. pHJ $_{\alpha 1}$-4

pHJ $_{\alpha 1}$-4, shown in Table 1, was constructed substantially as described in Example 2, above, and shown to produce HJ2 as 0.5-1% of total cellular protein.

## Ex. 9. p $_{\alpha 1}$-A

pASK is a plasmid which comprises the PL promoter and a gene for E. coli galactokinase (galK) including the cII ribosome binding site. It was constructed by placing the cII ribosome binding region of pAS1 adjacent to a galK coding sequence into which a BamHI linker had been inserted between the ATG and the second codon of the galK coding sequence. Transformation of E. coli C600 (cI857) resulted in transformants which when plated on an indicator plate comprising MacConkey galactose caused the agar to redden around the transformants.

The 45 base pair BamHI-BclI fragment from the native AAT coding sequence was inserted into the BamHI site, resulting in a translational fusion of the AAT N-terminus and the galK coding sequence. This plasmid, pAAT galK, upon transformation into E. coli C600 (cI857), did not cause the indicator plates to turn red. Western blot analysis confirmed that such transformants produced the hybrid protein in amounts equal to roughly 0.3 to 0.5 percent of the amounts of galK expressed by cells transformed with pASK.

In order to generate mutants, pAATgalK was treated with hydroxylamine in vitro and, in a separate experiment, was passaged through a mutator cell line, namely; mut T or mut D (See Miller, ed., Experiments in Molecular Genetics.) E. coli C600 (cI857) was transformed with the plasmid which had been subjected to the mutagenic conditions and plated onto indicator plates. Several red colonies were isolated and analyzed by Western blot analysis for levels of the AAT-galK hybrid protein. One "up-mutant" produced about 10 to 20-fold greater amounts of the hybrid than the original pAATgalK transformants. When this gene was sequenced, it was found that a single point mutation had occurred in the 2nd codon, changing said codon from GAT (aspartic acid) to AAT (asparagine).

Plasmid $p_{\alpha 1}$-A was then constructed by insertion of synthetic oligonucleotides (See Table 1, above) into pHJ $_{\alpha 1}$-1 and subsequently into pOTS $_{\alpha 1}$, as described in Example 6. E. coli C600 (cI857) transformed with $P_{\alpha 1}$-A produced about 10 times more of the variant AAT than the amount of AAT from pHJ$_{\alpha 1}$-1.

### Ex. 10. pHJ$_{\alpha 1}$-7

A synthetic oligonucleotide containing the single nucleotide change in $P_{\alpha 1}$-A and the three changes in pSYN7R was inserted into pHJ $_{\alpha 1}$-1. Expression of the resulting variant AAT HJ7 was determined by Western blot analysis to be 5- to 10-fold greater than Syn 7, and about 30-50-fold greater than from pHJ $_{\alpha 1}$-1.

### Ex. 11. pOTS$_{\alpha 1}$-7

A BstXI fragment of pHJ$_{\alpha 1}$-7, which comprises regions upstream of the variant AAT coding sequence through a portion of the AAT coding sequence downstream of the 5' mutations, was inserted in place of a BstXI fragment in pOTS$_{\alpha 1}$. The sites in both vectors are at identical positions with respect to the AAT gene. The resultant construct, pOTS$_{\alpha 1}$-7, was transformed into E. coli strain AR120. Induction resulted in expression of HJ7 in amounts equal to about 15% of total cellular protein, or about 150- to 200-fold greater than AAT expressed by pHJ$_{\alpha 1}$-1.

### Ex. 12. $p_{\alpha 1}$-AG

A synthetic oligonucleotide containing the single nucleotide change present in $P_{\alpha 1}$-A and a C to G change in the 3rd position of the 3rd codon (proline) (see Table 1) was inserted into pOTS$_{\alpha 1}$ as described in Example 9. E. coli (c600 cI857) transformed with p$_{\alpha 1}$-AG produced about 20 times more of the variant AAT, HJ7, than the amount of AAT produced from pHJ$_{\alpha 1}$-1.

## Ex. 13. p$_{\alpha 1}$-AG cro

The expression vector, pRDNS, was constructed as follows: The vector pMG27N (see Example 2) was restricted with AvaI and the 423 bp AvaI-XhoI and 774 bp XhoI-AvaI fragments from pOTS were inserted. The resulting vector, pMG27N-S was cut with BamHI and ligated with the BamHI fragment from pAS1 Δ EH801 which contains the NS1 gene (Young et al., Proc. Nat'l. Acad. Sci. USA 60: 6105-6109 1983)). This vector, pMG27N-SNS1 was cut at its unique NdeI site, treated with mung bean nuclease and religated. This vector, pMG27N-SNS1-MB was cut with SalI and NcoI. Between these sites was inserted a synthetic oligonucleotide which encoded for a mutant cro ribosome binding site (rbs). The mutations introduced in this rbs (CA instead of TG at positions -3 and -2 respectively with respect to the ATG) allowed us to introduce unique NdeI and SalI sites adjacent to the ATG. The Shine-Dalgarno sequence and distance to the ATG are identical to those in the wild type cro rbs. The wild type AAT sequence was moved from pHJ$_{\alpha 1}$-1 to pRDNS to place the coding sequence under PL control and the cro ribosome binding site. A BstXI fragment from the plasmid was then inserted between BstXI cut pOTS$_{\alpha 1}$ as in Example 11. The resulting plasmid, pRDNS$_{\alpha 1}$-1 expresses AAT at levels 3 times greater than the plasmid pOTS$_{\alpha 1}$-1 even though no changes were made in the gene coding sequence.

The variant AAT coding sequence from p$_{\alpha 1}$-AG was inserted into pRDNS to place the coding sequence under control of P$_L$ and the cro ribosome binding site. A BstXI fragment was cloned into pOTS $_{\alpha 1}$ as described above. The resulting plasmid was $p_{\alpha 1}$-AGcro. E. coli transformants containing this plasmid produce the variant AAT in amounts 3 times greater than transformants with $p_{\alpha 1}$-AG and about 60 times greater than the amount of AAT from pHJ$_{\alpha 1}$-1.

### EX. 14 $p_{\alpha 1}$-Acro

The variant AAT coding sequence from $p_{\alpha 1}$-A was inserted into pRDNS to place the coding sequence under control of PL and the cro ribosome binding site. A BstXI fragment switch was again performed with pOTS $_{\alpha 1}$. The resulting plasmid is $p_{\alpha 1}$-Acro. E. coli transformants with this plasmid produced HJ7 in amounts about 3-fold greater than transformants with $p_{\alpha 1}$-A.

The above description and examples are fully illustrative of the invention and of preferred embodiments thereof. The invention, however, is not limited to the precise constructions disclosed herein but, rather, encompasses all modifications thereof within the scope of the following claims.

<u>Claims for the Contracting States</u>
<u>BE - CH - DE - FR - GB - IT - LI - LU - NL - SE</u>

1. A DNA molecule comprising a mutant alpha-1-antitrypsin (AAT) coding sequence wherein the mutation comprises an alteration of the AAT coding sequence within codons 1-15, provided that the mutation is other than or additional to substitution of the codon ATG for the first codon of the native coding sequence.

2. The DNA molecule of claim 1 wherein the mutation comprises substitution of third base pair positions which do not affect primary structure of the variant AAT expressed thereby, deletion of one or more codons, or both.

3. The DNA molecule of claim 2 wherein the coding sequence is altered within codons 1-10.

4. The DNA molecule of claim 2 wherein the coding sequence comprises the N-terminal alterations present in $pHJ_{\alpha 1}-2$, $pHJ_{\alpha 1}-3$, $pOTS_{\alpha 1}Bcl$, $pHJ_{\alpha 1}-4$, $pHJ_{\alpha 1}-5$, $pHJ_{\alpha 1}-6$, pSyn7, pSyn7R, $p_{\alpha 1}-A$ or $pHJ_{\alpha 1}-7$.

5. The DNA molecule of claim 2 which is $pHJ_{\alpha 1}-2$, $pOTS_{\alpha 1}-3$, $pHJ_{\alpha 1}-4$, $pHJ_{\alpha 1}-5$, $pHJ_{\alpha 1}-6$, $pOTS_{\alpha 1}Bcl$, pSyn7, pSyn7R, $p_{\alpha 1}-A$, $pHJ_{\alpha 1}-7$, $pOTS_{\alpha 1}-7$, $p_{\alpha 1}-Acro$, $P_{\alpha 1}-AG$, $p_{\alpha 1}-AGcro$, $pOTS_{\alpha 1}-5$ or $pOTS_{\alpha 1}-6$.

6. The DNA molecule of claim 2 wherein the mutation comprises deletion of about the first 10 codons for native AAT or of about the first 5 codons for native AAT.

7. The DNA molecule of claim 6 which is $pHJ_{\alpha 1}-6$ of $pHJ\alpha_1-7$.

8. The DNA molecule of claims 1, 2, 3, 4, or 6 which is a bacterial expression vector having the mutant alpha-1-antitrypsin coding sequence operatively inserted therein.

9. The DNA molecule of claim 8 which is an E. coli expression vector.

10. The DNA molecule of claim 9 wherein the expression vector comprises the regulatory region of pAS1, pCVQ2, or pRDNS.

11. A variant AAT protein having anti-trypsin and anti-elastase activity comprising an amino acid sequence altered within positions 1-15, provided that the mutation is other than or additional to substitution of a methionine residue in the first position of native AAT.

12. The variant AAT of claim 11 which is deleted in the first 15 amino acids or in the first 10 amino acids or in the first 5 amino acids.

13. The variant AAT of claim 11 which is AATD15, AATD10 or AATD5.

14. The variant AAT of claim 11 which is AATD5.

15. The variant AAT of claim 11 which is HJ2, HJ7 or Syn 7.

16. A bacterium transformed with the DNA molecule of any of claims 1 through 7.

17. The bacterium of claim 16 which is an E. coli.

18. A bacterium transformed with the DNA molecule of claim 8.

19. The bacterium of claim 18 which is an E. coli.

20. An E. coli transformed with the DNA molecule of claim 9.

21. An E. coli transformed with the DNA molecule of claim 10.

22. A method of producing a polypeptide having the anti-trypsin and anti-elastase activities of AAT, which comprises culturing bacteria of claim 16 under permissive conditions, such that the polypeptide is expressed in recoverable quantity, and isolating the polypeptide from said culture.

23. The method of claim 22 wherein the bacteria are E. coli.

24. A method of producing a polypeptide having the anti-trypsin and anti-elastase activities of AAT which comprises culturing bacteria of claim 18 under permissive conditions, such that the polypeptide is expressed in recoverable quantity, and isolating the polypeptide from said culture.

25. The method of claim 24 wherein the bacteria are E. coli.

26. A method of producing a polypeptide having the anti-trypsin and anti-elastase activities of AAT which comprises culturing the E. coli of claim 20 under permissive conditions, such that the polypeptide is expressed in recoverable quantity, and isolating the polypeptide from said culture.

27. A method or producing a polypeptide having the anti-trypsin and anti-elastase activity of AAT which comprises culturing the E. coli of claim 21 under permissive conditions, such that the polypeptide is expressed in recoverable quantity, and isolating the polypeptide from said culture.

28. A pharmaceutical composition for inhibiting elastase activity in the lungs of an animal comprising the variant AAT protein of claim 11, 12, 13, 14 or 15 and a pharmaceutically acceptable carrier.

29. The pharmaceutical composition of claim 28 prepared for intravenous administration.

30. A method of identifying a mutant coding sequence for a polypeptide of interest which is transcribed and translated in an amount greater than the native coding sequence which comprises (i) ligating the whole or a portion of the native coding sequence to a coding sequence for a selectable phenotype, transforming a suitable host therewith and quantitating expression of the resulting translational fusion, (ii) mutagenizing the whole or the portion of the coding sequence for the polypeptide of interest, (iii) assaying for increased expression of the mutant translational fusion over the expression of the original translational fusion; (IV) determining the nucleotide sequence of the coding sequence for the polypeptide of interest in fusions displaying increased expression in (iii) and selecting those having a mutation in the coding sequence for the polypeptide of interest, (v) preparing a coding sequence identical to the native coding sequence for the product of interest except that it contains one or more of the mutation(s) identified in the mutant coding sequence and transforming a suitable host therewith, and (vi) selecting clones of transformants from (v) in which the mutant coding sequence is transcribed and translated in amounts greater than the native coding sequence.

31. The method of claim 30 wherein the whole or portion of the native coding sequence employed in step (i) is a portion coding for N-terminal amino acids and is ligated upstream of the coding sequence for the selectable phenotype.

32. The method of claim 31 wherein the native coding sequence is a sequence for human alpha-1-antitrypsin and the host is an E. coli.

33. The method of claim 32 wherein, in step (i), a portion of the AAT coding sequence which codes for the first fifteen amino acids of AAT is ligated to the coding sequence for a selectable phenotype which is a coding sequence for galactokinase or beta-galactosidase and the assay for increased expression comprises culturing the transformants on an indicator plate.

Claims for the Contracting States : AT - GR - SP

1. A method of producing a polypeptide having the anti-trypsin and anti-elastase activities of AAT, which comprises culturing a bacterium transformed with a DNA molecule comprising a mutant alpha-1- antitrypsin (AAT) coding sequence wherein the mutation comprises an alteration of the AAT coding sequence within codons 1-15, provided that the mutation is other than or additional to substitution of the codon ATG for the first codon of the native coding sequence under permissive conditions, such that the polypeptide is expressed in recoverable quantity, and isolating the polypeptide from said culture.

2. A method of producing a polypeptide having the anti-trypsin and anti-elastase activities of AAT, which comprises culturing a bacterium transformed with a DNA molecule according to claim 1 wherein the mutation comprises substitution of third base pair positions which do not affect primary structure of the variant AAT expressed thereby, deletion of one or more codons, or both under permissive conditions, such that the polypeptide is expressed in recoverable quantity, and isolating the polypeptide from said culture.

3. A method of producing a polypeptide having the anti-trypsin and anti-elastase activities of AAT, which comprises culturing a bacterium transformed with a DNA molecule according to claim 2 wherein the coding sequence is altered within codons 1-10 under permissive conditions, such that the polypeptide is expressed in recoverable quantity, and isolating the polypeptide from said culture.

4. A method of producing a polypeptide having the anti-trypsin and anti-elastase activities of AAT, which comprises culturing a bacterium transformed with a DNA

molecule according to claim 2 wherein the coding sequence comprises the N-terminal alterations present in $pHJ_{\alpha 1}-2$, $pHJ_{\alpha 1}-3$, $pOTS_{\alpha 1}Bcl$, $pHJ_{\alpha 1}-4$, $pHJ_{\alpha 1}-5$, $pHJ_{\alpha 1}-6$, pSyn7, pSyn7R, $P_{\alpha 1}-A$ or $pHJ_{\alpha 1}-7$ under permissive conditions, such that the polypeptide is expressed in recoverable quantity, and isolating the polypeptide from said culture.

5. A method of producing a polypeptide having the anti-trypsin and anti-elastase activities of AAT, which comprises culturing a bacterium transformed with a DNA molecule according to claim 2 which is $pHJ_{\alpha 1}-2$, $pOTS_{\alpha 1}-3$, $pHJ_{\alpha 1}-4$, $pHJ_{\alpha 1}-5$, $pHJ_{\alpha 1}-6$, $pOTS_{\alpha 1}Bcl$, pSyn7, pSyn7R, $P_{\alpha 1}-A$, $pHJ_{\alpha 1}-7$, $pOTS_{\alpha 1}-7$, $P_{\alpha 1}-Acro$, $P_{\alpha 1}-AG$, $P_{\alpha 1}-AGcro$, $pOTS_{\alpha 1}-5$ or $pOTS_{\alpha 1}-6$ under permissive conditions, such that the polypeptide is expressed in recoverable quantity, and isolating the polypeptide from said culture.

6. A method of producing a polypeptide having the anti-trypsin and anti-elastase activities of AAT, which comprises culturing a bacterium transformed with a DNA molecule according to claim 2 wherein the mutation comprises deletion of about the first 10 codons for native AAT or of about the first 5 codons for native AAT under permissive conditions, such that the polypeptide is expressed in recoverable quantity, and isolating the polypeptide from said culture.

7. A method of producing a polypeptide having the anti-trypsin and anti-elastase activities of AAT, which comprises culturing a bacterium transformed with a DNA molecule according to claim 6 which is $pHJ_{\alpha 1}-6$ or $pHJ\alpha_1-7$ under permissive conditions, such that the polypeptide is expressed in recoverable quantity, and isolating the polypeptide from said culture.

8. A method of producing a polypeptide having the anti-trypsin and anti-elastase activities of AAT, which comprises culturing a bacterium transformed with a DNA molecule according to claims 1, 2, 3, 4, or 6 which is a bacterial expression vector having the mutant alpha-1-antitrypsin coding sequence operatively inserted therein under permissive conditions, such that the polypeptide is expressed in recoverable quantity, and isolating the polypeptide from said culture.

9. A method of producing a polypeptide having the anti-trypsin and anti-elastase activities of AAT, which comprises culturing a bacterium transformed with a DNA molecule according to claim 8 which is an E. coli expression vector under permissive conditions, such that the polypeptide is expressed in recoverable quantity, and isolating the polypeptide from said culture.

10. A method of producing a polypeptide having the anti-trypsin and anti-elastase activities of AAT, which comprises culturing a bacterium transformed with a DNA molecule according to claim 9 wherein the expression vector comprises the regulatory region of pAS1, pCVQ2, or pRDNS under permissive conditions, such that the polypeptide is expressed in recoverable quantity, and isolating the polypeptide from said culture.

11. The method of any of claims 1 to 10 wherein the bacterium is E. coli.

12. A method of identifying a mutant coding sequence for a polypeptide of interest which is transcribed and translated in an amount greater than the native coding sequence which comprises (i) ligating the whole or a portion of the native coding sequence to a coding sequence for a selectable phenotype, transforming a suitable host therewith and quantitating expression of the resulting translational fusion, (ii) mutagenizing the whole or the portion of the coding sequence for the polypeptide of interest, (iii) assaying for increased expression of the mutant translational fusion over the expression of the original translational fusion; (IV) determining the nucleotide sequence of the coding sequence for the polypeptide of interest in fusions displaying increased expression in (iii) and selecting those having a mutation in the coding sequence for the polypeptide of interest, (v) preparing a coding sequence identical to the native coding sequence for the product of interest except that it contains one or more of the mutation(s) identified in the mutant coding sequence and transforming a suitable host therewith, and (vi) selecting clones of transformants from (v) in which the mutant coding sequence is transcribed and translated in amounts greater than the native coding sequence.

13. The method of claim 12 wherein the whole or portion of the native coding sequence employed in step (i) is a portion coding for N-terminal amino acids and is ligated upstream of the coding sequence for the selectable phenotype.

14. The method of claim 13 wherein the native coding sequence is a sequence for human alpha-1-antitrypsin and the host is an E. coli.

15. The method of claim 14 wherein, in step (i), a portion of the AAT coding sequence which codes for the first fifteen amino acids of AAT is ligated to the coding sequence for a selectable phenotype which is a coding sequence for galactokinase or beta-galactosidase and the assay for increased expression comprises culturing the transformants on an indicator plate.